Europäisches Patentamt

European Patent Office

Office européen des brevets

(11) Publication number: **0 137 538**
**B1**

(12) **EUROPEAN PATENT SPECIFICATION**

(45) Date of publication of patent specification: **14.03.90**

(21) Application number: **84201269.2**

(22) Date of filing: **04.09.84**

(51) Int. Cl.⁵: **C 09 K 7/06,** E 21 B 43/25, C 09 D 11/00

(54) Biopolymer formulations and processes for preparing them.

(30) Priority: **09.09.83 GB 8324236**
**11.05.84 GB 8412053**

(43) Date of publication of application:
**17.04.85 Bulletin 85/16**

(45) Publication of the grant of the patent:
**14.03.90 Bulletin 90/11**

(84) Designated Contracting States:
**AT BE CH DE FR GB IT LI LU NL SE**

(56) References cited:
EP-A-0 049 012
EP-A-0 128 661
EP-A-0 130 647
FR-A-2 429 236
GB-A-2 115 430
US-A-3 826 771
US-A-4 392 917

The file contains technical information submitted after the application was filed and not included in this specification

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Bleeker, Jan Jacob**
**Badhuisweg 3**
**NL-1031 CM Amsterdam (NL)**
Inventor: **Lammers, Jan Hendrik**
**Badhuisweg 3**
**NL-1031 CM Amsterdam (NL)**
Inventor: **Roest, Jacob Bernard**
**Badhuisweg 3**
**NL-1031 CM Amsterdam (NL)**
Inventor: **Eckert, Rudolf Josef Albrecht**
**Badhuisweg 3**
**NL-1031 CM Amsterdam (NL)**

(74) Representative: **Bennett, David Arthur Horder et al**
**4, York Road**
**London SE1 7NA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to a biopolymer formulation and in particular to a polysaccharide-containing water in oil emulsion, a process for preparing such emulsions and a process for concentrating it.

Polysaccharides play an important role as basic materials for the food industry. However an increasing interest for other applications is being developed, such as in printing ink, wherein they act as thickeners. In particular because of their ability to thicken these polysaccharides are now also applied in enhanced oil recovery operations and also appear to be very useful in drilling fluids and completion fluids at oil exploration sites. Since highly concentrated aqueous solutions still only contain up to ten percent by weight of these biopolymers transport over long distances is not very economic especially when large amounts, required for enhanced oil recovery operations, have to be transported. Furthermore highly concentrated aqueous biopolymer solutions are difficult to handle because of their high viscosity which may cause pumping problems and losses when discharging containers. Therefore methods are being developed to obtain concentrates containing a higher percentage of the desired material in order to decrease the transport costs and improve the handability. GB—A—2018300 describes dispersions in oil of thickening agents such as xanthan polysaccharides which can reach concentrations of up to 40% wt of the thickening agent. In order to prepare such dispersion one uses the biopolymer in powered form as a starting material.

However handling of powder is not entirely without difficulties. Dust problems inherent to the handling of powders and the fact that the powder has the tendency to clot when brought in contact with the dispersing liquid make the powder-form less attractive.

US Patent No. 4,392,917 discloses an amphoteric water-in-oil self-inverting polymer emulsion which contains a copolymer of (1) a nonionic vinyl monomer and (2) an amphoteric vinyl monomer or a terpolymer of (1) a nonionic vinyl monomer, (2) an anionic vinyl monomer and (3) a cationic vinyl monomer in the aqueous phase, a hydrocarbon oil for the oil phase, a water-in-oil emulsifying agent and an inverting surfactant. This water-in-oil emulsion is prepared by dissolving the required members in the water phase, dissolving the emulsifying agent in the oil phase, emulsifying the water phase in the oil phase to prepare a water-in-oil emulsion, homogenizing the water-in-oil emulsion, polymerizing the monomers dissolved in the water phase of the water-in-oil emulsion to obtain the copolymer or terpolymer and then adding the self-inverting surfactant to obtain an amphoteric water-in-oil self-inverting water-in-oil emulsion. The emulsion is useful in papermaking, treatment of sewage and industrial wastes, drilling muds and secondary and tertiary recovery of petroleum by water flooding.

US Patent No. 3,826,771 discloses a stable water-in-oil polymer emulsion which contains dispersed therein finely-divided particles of water-soluble vinyl addition polymer in the concentration of at least 20% based on emulsion, and the preparation of such emulsions by:

A. using as a reaction mixture:

(1) water which contains dissolved therein a water-soluble vinyl addition monomer thereby producing a monomer phase, having a concentration of from 75 to 95% by weight of the emulsion;

(2) an inert hydrophobic liquid ranging between 5 and 25% by weight of the emulsion;

(3) water-in-oil emulsifying agent in a concentration of 0.1 to 15%; and

(4) a free radical initiator whereby a water-in-oil emulsion is formed; and

B. heating the emulsion under free radical conditions to polymerise the water-soluble vinyl addition monomer containing emulsion thereby producing a stable water-in-oil emulsion which contains dispersed therein finely-divided particles of a water-soluble vinyl addition polymer.

It has now been found that biopolymer in oil emulsions can be prepared having a concentration of polysaccharide which is much higher than 40% wt and which emulsions appear to be surprisingly stable. The present emulsions can suitably be prepared from aqueous solutions of polysaccharides having a concentration of up to about ten percent by weight.

Furthermore, it is a characterizing feature of the present invention that polysaccharides are substantially present in the emulsion in the form of globules or droplets which are still in a more or less swollen state and which, as is believed, makes them dissolve into water more easily than substantially water-free particles. This characteristic of the present emulsion is especially in enhanced oil operations and when the emulsion is used e.g. in drilling muds and in well-completion fluids—very important, because for those applications free-flowing and easy water dissolvable polysaccharide formulations are required. In conventional dispersions of polysaccharides derived from dry polysaccharide powder, the biopolymer particles are substantially water-free and therefore dissolve more slowly into water. They tend to stick together thus forming clots. Furthermore filterability problems can arise when dispersions are rehydrated.

Accordingly the present invention provides a biopolymer water-in-oil emulsion of a polysaccharide biopolymer which comprises 1—70% wt of a microbially produced polysaccharide in the form of water-swollen particles, 10—60% wt hydrophobic liquid, 5—60% wt water; 1—25% wt emulsifier; and 1—15% wt of a stabiliser selected from polyalkylmethacrylates, polyalkylacrylates, copolymers of alkylacrylates with vinyl pyridines, copolymers of alkylmethacrylates with vinyl pyridines, and combinations thereof.

Preferably the biopolymer water-in-oil emulsion comprises 30—65% wt polysaccharide, 15—40% wt hydrophobic liquid, 5—25% wt water, 5—25% wt emulsifier, and 1—15% wt of the stabiliser. The emulsion according to the present invention may optionally contain mixtures of two or more emulsifying agents together with mixtures of two or more stabilizing agents.

Emulsifiers can suitably be selected from non-ionic like alkylphenol-ethoxylates, ethoxylated long chain fatty acids, polyethylene glycol mono-oleates, polyethyleneglycol-dioleates, polyethyleneglycol-laurates, sorbitan fatty acid esters such as the "Span" (registered trade mark) emulsifiers, which are emulsifiers of the German company Atlas Chemie GmbH, especially "Span 80" which is sorbitan mono-oleate and ethoxylated linear alcohols like "Dobanol", a registered trade mark of Shell. "Dobanol" stands for a series of synthetic primary fatty alcohols having 9 to 15 carbon atoms. Further examples of suitable emulsifiers are the product from the company AKZO i.e. sorbitan-monolaurate, sorbitan-mono-palmitate and sorbitan-mono-stearate sold under respectively the registered trade marks "Armotan" ML, "Armotan" MP an "Armotan" MS. Also the ethoxylated versions of the above compounds are suitable. Those are sold under the registered trademarks "Armotan" PML 20, "Armotan" PMP 20 and "Armotan" PMS 20. Further combinations of the non-ethoxylated and ethoxylated emulsifiers might be used. Sorbitan fatty acids can suitably be combined with poly-oxyethylene sorbitan fatty acid esters. Other examples of non-ionic emulsifiers can be found in the group of the ethoxylated octyl- and nonylphenols and the ethoxylated alcohols.

Cationic emulsifiers like ethoxylated fatty amines and primary fatty amines or other amine type of emulsifiers which are oil soluble can also be applied for preparing a biopolymer in oil emulsion. "Ethomenes" and "Armenes" of Akzo Chemie Nederland b.v. based on tallow, cocco, soya and oleic oil such as "Ethomeen T12" (registered trade mark) (bis(2-hydroxy)-tallowamine), "Armeen HT" (registered trade mark) (tallowamine) are very suitable examples.

Preferably the emulsifier is selected from the group consisting of sorbitan-mono-oleate, sorbitan mono-laurate, ethoxylated alkylphenol, sorbitan-mono-palmitate, sorbitan-mono-stearate, poly-isobutylene - maleic - anhydride - triethylene-tetraamine, bis(2-hydroxyethyl) tallowamine, tallowamine or combinations thereof.

A preferred combination of non-ionic emulsifiers is a mixture of sorbitan mono-oleate, sorbitan mono-laurate and an ethoxylated alkylphenol, or a mixture of poly-isobutylene-maleic-anhydride-triethylene-tetraamine and bis(2-hydroxyethyl)tallowamine.

Stabilizers which appear to be highly suitable are selected from the group consisting of polyalkylmethacrylates, polyalkylacrylates, copolymers of alkylacrylates with vinyl pyridines and copolymers of alkylmethacrylates with vinyl pyridines.

Their composition and their molecular weights may vary considerably. Typical examples are:

a copolymer of C20/22-acrylate and minor amounts of C16/18-acrylate having a weight average molecular weight (Mw) of 200.000—500.000 and a number average molecular wight (Mn) of 49.000—76.000 as a ca. 50% w solution in toluene or xylene;

the toluene solution (40% w of active matter) of a copolymer of C20/22-acrylate (89.4% w) and 4-vinyl pyridine (10.6% w) having a Mw=70.000 to 270.000 and Mn=25.000—55.000;

a 40% w concentrate in an HVI-oil 60 of a copolymer of C16/18-alkylmethacrylate (44.5% w), "Dobanol" 25-methacrylate (10.0% w) and "Linevol" 911-methacrylate (45.5% w) with Mw=40.000—600.000 and Mn=20.000—150.000 ("Dobanol" and "Linevol" are registered trade marks);

a copolymer of C16/18-alkylmethacrylate (21.6% w), "Dobanol" 25-methacrylate (54.9%), "Linevol" 911-methacrylate (18.1% w) and 4-vinyl pyridine (5.4% w) with molecular weight Mw=155.000—200.000 and Mn=60.000—80.000 as a ca. 55% w concentrate in a mineral oil;

a copolymer of C16/18-alkylmethacrylate (4.0% w), "Dobanol" 25-methacrylate (76.7% w), "Linevol" 911-methacrylate (8.2% w) and methyl methacrylate (11.1% w) with Mw=105.000—140.000 and Mn=40.000—60.000 as a 60—65% w solution in a mineral oil;

copolymers of C16/18-alkylmethacrylate (30.1% w), "Dobanol" 25 methacrylate (47.8% w) and 2- or 4-vinyl pyridine (22.1% w) with Mw=40.000—65.000 and Mn of ca. 22.000 as 44% w solutions in xylene.

"Dobanol 25" is a mixture of primary linear C12/15-alcohols. "Linevol 911" is a mixture of primary C9/11-alcohols.

Other additives, such as dispersing agents can suitably be present in the water-in-oil emulsion according to the invention in order to enhance the dispersibility of the formulation. Hydration time is shortened and the viscosity development is enhanced and is faster when dispersing agents are used.

Suitable dispersing agents are hydrophilic surfactants, known to those skilled in the art, like ethoxylated alkylphenols and ethoxylated alcohols sulfonates. Preferred dispersing agents are selected from the group consisting of "Nonidet-NP50", "Dobanol" or combinations thereof. "Nonidet-NP50" is nonylphenol ethoxylate ("Nonidet" is a registered trade mark).

Preferably the polysaccharide is derived from micro-organisms like Xanthomonas campestris NCIB11854 and Pseudomonas sp. NCIB11592 since these microorganisms are commercially readily available.

However the polysaccharide may also be suitably produced by other micro-organisms like Xanthomonas phaseoli, Xanthomonas carotae, Xanthomonas, begomase, Xanthomonas meanae, Xanthomonas malvacearum, Xanth-

*omonas vesicatoria, Xanthomonas papavericola, Xanthomonas incanae, Xanthomonas translucens, Rhizobium meliloti, Alcaligenes faecalis* var. *myxogenes, Agrobacterium tumefaciens, Agrobacterium radiobacter,* and *Agrobacterium rhizogenes.*

The polysaccharides is suitably applied in the form of an aqueous solution which may be obtained by ultrafiltrating and optionally concentrating of a fermentation broth, as described in EP—A—49012, and/or enzyme treatment, as descirbed in EP—A—39962, and/or enzyme/surfactant treatment, as described in EP—A—78556, and/or contacting the fermentation broth with particles of solid siliceous material at an adsorption-enhancing pH, as described in GB—A—1598594.

Preferably the polysaccharide is derived from a clarified and aqueous bacterial optionally concentrated fermentation broth, substantially free from cellular debris and containing 3—25% wt of polysaccharides. An emulsion prepared from this starting material may therefore contain as low as 1% wt polysaccharide.

The hydrophobic liquid is suitably an unsubstituted and/or substituted hydrocarbon liquid which encompasses aliphatic and aromatic compounds.

For instance hydrocarbons like a mineral oil, a kerosine or a naphtha are suitable so are organic hydrocarbons like benzene, xylene and toluene. An oilphase on the basis of branched hydrocarbons for instance on the basis of isoparaffins is particularly suitable. Further water immiscible alcohols having 8 to 20, preferably 8 to 12 carbonatoms, vegetable oils such as cornoil, peanut oil, soybean oil, and sunflower oil, esteralcohols, polyolethers or other heteroatoms containing compounds for instance siliconoils may suitably be used as well. Certain halogenated hydrocarbons have also been found useful.

Preferably the hydrophobic liquid is a white spirit or a mixture of white spirits.

A preferred emulsion according to the present invention comprises 30—60% wt polysaccharide derived from *Xanthomonas campestris* NCIB 11854 and/or *Pseudomonas* sp. NCIB 11592, 25—45% wt of white spirit, 5—25% water and 5—20% of a mixture of sorbitan mono-oleate and sorbitan mono-laurate and/or a mixture of polyisobutylene-maleic-anhydride-triethylenetetraamine and bis(2-hydroxyethyl)tallowamine.

The emulsions according to the present invention can be prepared using emulsification techniques known in the art. The present invention further provides a biopolymer emulsion which is obtained after a biopolymer emulsion as hereinbefore described has been subjected to a shear treatment in high shear devices or to milling in a colloid mill whereby the size of the polysaccharide globules or droplets is substantially reduced and a finer emulsion is obtained. Besides emulsifiers the emulsions according to the present invention may also suitably contain stabilizing agents, suspending agents and surfactants to enhance e.g. stability and hydration, wettability of the polymer droplets upon dissolution in water. Furthermore the viscosity of the emulsion can be adjusted to ones wishes by adding oil viscosifiers, which are suitably oil-soluble polymers. Also thickeners can be employed to increase the viscosity of the hydrophobic liquid or oil and to improve storage stability. Suitable examples of thickeners include organophilic clays like the bentonites or montmorillonites which are amine treated to make them organophilic. Other thickeners which may be suitably employed are colloidal silica, fumed silica and the like. Commercially available thickener are "Cab-o-Sil" (by Cabot) (registered trade mark) and Thixogel (by Sud-chemie) (registered trade mark). Additional thickeners include metallic soaps, such as the metal salts of higher monocarboxylic acids.

The present invention also relates to an aqueous system whenever thickened by the incorporation of a biopolymer emulsion as described hereinbefore. Such an aqueous system is for instance a printing ink.

Preferably the aqueous system is a flooding material which is used in enhanced oil recovery operations. Still further the present invention provides a process for concentrating a biopolymer emulsion as hereinbefore described which comprises removing water from the emulsion by evaporation under reduced pressure and at elevated temperature in order to get a higher concentration of biopolymers in the water in oil emulsion. Preferably the process is continued until the concentration of the biopolymers is in the range of 35—45% wt since it appears that at a concentration in that range the emulsion is most easily hydrated than at higher concentrations. However if desired further concentration towards 70% wt can be achieved.

The emulsion may also be concentrated by other evaporation techniques like azeotropic distillation, vacuum drying, spray drying and microwave drying.

Still preferably the concentration is achieved by film-evaporation which may suitably be carried out with a falling or climbing film-evaporation and/or wiped film evaporation.

Most preferably evaporation is carried out in a rota vapor or a wiped film evaporator. In the present process the reduced pressure is preferably in the range of 1—50 mm Hg (133—6666 Pa) and the temperature is in the range of 40—120°C. The residence time of the biopolymer emulsion in the film-evaporation equipment is short and suitable in the range of 1—20 min since longer residence times at higher temperatures might harm the biopolymer.

If the biopolymer emulsion appears to contain relatively coarse (0.1—5 mm) biopolymer droplets which may not be desired than the biopolymer emulsion is preferably subjected to a shear treatment or processed through a colloid mill in order to obtain an emulsion containing finer biopolymer droplets.

The present invention will be further described with reference to the following Example.

Example

An emulsion was prepared by mixing 4.0 g "Span 20", 1.0 g "Nonidet-NP50", 1.0 g polymethacrylate and 8.0 g "Ondina 15" high-boiling hydrophobic solvent ("Ondina" is a registered trade mark) and 186 g "Shellsol TD" low-boiling hydrophobic solvent ("Shellsol" is a registered trade mark). 200 g Xanthan biopolymer aqueous concentrate 9% wt derived from Xanthomonas campestris NCIB 11854 was added, while using an ultra turrax mixer for 1 min. After concentration in an evaporating device an emulsion with a concentration of biopolymer of 55% wt was obtained which appears to be stable at 20°C and 60°C for at least 12 weeks. Furthermore this emulsion appears to hydrate extremely fast and complete and is in this respect far superior to a typical Xanthan powder like "Kelzan XCD" ("Kelzan" is a registered trade mark).

## Claims

1. Water-in-oil emulsion of a polysaccharide biopolymer which comprises 1—70% wt of a microbially produced polysaccharide in the form of water swollen particles: 10—60% wt of a hydrophobic liquid; 5—60% wt water; 1—25% wt of an emulsifier, and 1—15% wt of a stabiliser selected from polyalkylmethacrylates, polyalkyacrylates, copolymers of alkylacrylates with vinyl pyridines, copolymers of alkylmethacrylates with vinyl pyridines, and combinations thereof.

2. Emulsion as claimed in claim 1 wherein the emulsifier is selected from sorbitan mono-oleate, sorbitan monolaurate, sorbitan monopalmitate, sorbitan monostearate, ethoxylated alkylphenol, polyisobutylene/maleic anhydride/triethylene tetraamine, bis-(2-hydroxyethyl) tallowamine, tallowamine or combinations thereof.

3. Emulsion as claimed in claim 2 wherein the emulsifier is a mixture of sorbitan mono-oleate, sorbitan monolaurate and an ethoxylated alkylphenol, or a mixture of polyisobutylene/maleic anhydride/triethylene tetraamine and bis (2-hydroxyethyl) tallowamine.

4. Emulsion as claimed in claim 1, 2 or 3 wherein the polysaccharide is derived from *Xanthomonas campestris* ·NCIB 11854 or *Pseudomonas* sp. NCIB 11592.

5. Emulsion as claimed in claim 1, 2, 3 or 4, wherein the polysaccharide is derived from a clarified and optionally concentrated aqueous bacterial fermentation broth substantially free from cellular debris and containing 3—25% wt polysaccharide.

6. Emulsion as claimed in any one of the preceding claims wherein the hydrophobic liquid is a white spirit or mixture of white spirits.

7. Emulsion as claimed in any one of the preceding claims comprising 30—65% wt polysaccharide, 5—25% wt water, 5—25% wt emulsifier, 15—40% wt hydrocarbon solvents, and 1—15% wt of the stabiliser.

8. Biopolymer emulsion obtained by subjecting a water-in-oil emulsion as claimed in any one of the preceding claims to a shear treatment or to milling in a colloid mill, whereby the size of the polysaccharide droplets is substantially reduced.

9. A process for concentrating an emulsion as claimed in any one of the preceding claims in which water is removed from the emulsion by evaporation under reduced pressure and at elevated temperature.

10. Process as claimed in claim 9 wherein the evaporation is carried out in a rota vapor or wiped film evaporator.

11. Concentrated biopolymer emulsion when produced by a process as claimed in claim 10.

12. An aqueous oil recovery flooding system when thickened by the incorporation of an emulsion as claimed in any one of claims 1—8 or 11.

## Patentansprüche

1. Wasser-in-Öl-Emulsion eines Polysaccharid-Biopolymers, umfassend 1 bis 70 Gew.-% eines mikrobiell erzeugten Polysaccharids in Form von in Wasser gequollenen Teilchen: 10 bis 60 Gew.-% einer hydrophoben Flüssigkeit; 5 bis 60 Gew.-% Wasser; 1 bis 25 Gew.-% eines Emulgiermittels und 1 bis 15 Gew.-% eines Stabilisators, ausgewählt aus Polyalkylmethacrylaten, Polyalkylacrylaten, Copolymeren von Alkylacrylaten mit Vinylpyridenen, Copolymeren von Alkylmethacrylaten mit Vinylpyridenen, und Kombinationen davon.

2. Emulsion nach Anspruch 1, wobei das Emulgiermittel ausgewählt ist aus Sorbitan-monooleat, Sorbitan-monolaurat, Sorbitan-monopalmitat, Sorbitan-monostearat, ethoxyliertem Alkylphenol, Polyisobutylen/Maleinsäure-anhydrid/Triethylentetraamin, Bis-(2-hydroxyethyl)talgfettamin, Telgfettamin oder Kombinationen davon.

3. Emulsion nach Anspruch 2, wobei das Emulgiermittel ein Gemisch ist aus Sorbitan-monooleat, Surbitan-monolaurat und einem ethoxylierten Alkylphenol, oder ein Gemisch von Polyisobutylen/Maleinsäure-anhydrid/Triethylen-tetraamin und Bis-(2-hydroxyethyl)talgfettamin.

4. Emulsion nach Anspruch 1, 2 oder 3, wobei das Polysaccharid abgeleitet ist von Xanthomonas campestris NCIB 11854 oder Pseudomonas sp. NCIB 11592.

5. Emulsion nach Anspruch 1, 2, 3 oder 4, wobei das Polysaccharid abgeleitet ist von einer geklärten und gegebenenfalls konzentrierten wäßrigen bakteriellen Fermentationsbrühe, die im wesentlichen frei ist von Zelltrümmern und 3 bis 25 Gew.-% Polysaccharid enthält.

6. Emulsion nach einem der vorangehenden Ansprüche, wobei die hydrophobe Flüssigkeit Testbenzin oder ein Gemisch von Testbenzin ist.

7. Emulsion nach einem der vorangehenden Ansprüche, umfassend 30 bis 65 Gew.-% Polysaccharid, 5 bis 25 Gew.-% Wasser, 5 bis 25 Gew.-% Emulgiermittel, 15 bis 40 Gew.-% Kohlenwasserstofflösungsmittel und 1 bis 15 Gew.-% Stabilisator.

8. Biopolymer-Emulsion, dadurch erhalten, daß man eine Wasser-in-Öl-Emulsion wie in einem der vorangehenden Ansprüche beansprucht, einer Scherbehandlung oder einer Mahlbehandlung in einer Kolloidmüle unterwirft, wobei die Größe der Polysaccharidtröpfchen wesentlich verringert wird.

9. Verfahren zum Konzentrieren einer Emulsion nach einem der vorangehenden Ansprüche, wobei Wasser von der Emulsion entfernt wird, durch Eindampfen unter vermischertem Druck und bei erhöhter Temperatur.

10. Verfahren nach Anspruch 9, wobei das Verdampfen in einem Rotationsverdampfer oder einem Abstreif-Filmverdampfer durchgeführt wird.

11. Konzentrierte Biopolymeremulsion, hergestellt nach einem Verfahren nach Anspruch 10.

12. Wäßriges System zum Fluten bei der Ölgewinnung, verdickt durch Einbau einer Emulsion nach einem der Ansprüche 1 bis 8 oder 11.

**Revendications**

1. Emulsion eau dans l'huile d'un biopolymère polysaccharide qui comprend 1—70% en poids d'un polysaccharide produit par voie microbienne sous forme de particules gonflées d'eau, 10—60% en poids de liquide hydrophobe, 5—60% en poids d'eau; 1—25% en poids d'émulsifiant; et 1—15% en poids d'un stabilisant choisi parmi les polyalkylméthacrylates, polyalkylacrylates, copolymères d'alkylacrylates avec des vinylpyridines, copolymères d'alkylméthacrylates avec des vinylpyridines, et leur combinaison.

2. Emulsion selon la revendication 1 dans laquelle l'émulsifiant est choisi parmi le monooléate de sorbitol, monolaurate de sorbitol, monopalmitate de sorbitol, monostéarate de sorbitol, alkylphénol éthoxylé, polyisobutylène-maléate de triéthylènetétraamine, bis-(2-hydroxyéthyl)(amine de tall-oil), amine de tall-oil ou leur combinaison.

3. Emulsion selon la revendication 2 dans laquelle l'émulsifiant est un mélange de monoo- léate de sorbitol, monolaurate de sorbitol et un alkylphénol éthoxylé, ou un mélange de polyisobutylènemaléate de triéthylène tétraamine et de bis(2-hydroxyéthyl)(amine de tall-oil).

4. Emulsion selon la revendication 1, 2 ou 3 dans laquelle le polysaccharide est produit par *Xanthomonas campestris* NCIB11854 ou *Pseudomonas* sp NCIB11592.

5. Emulsion selon la revendication 1, 2, 3 ou 4 dans laquelle le polysaccharide est produit par un bouillon de fermentation bactérien aqueux clarifié et éventuellement concentré exempt de débris cellulaires et comprenant 3—25% en poids de polysaccharide.

6. Emulsion selon l'une quelconque des revendications précédentes dans laquelle le liquide hydrophobe est un white spirit ou un mélange de white spirit.

7. Emulsion selon l'une quelconque des revendications précédentes comprenant 30—65% en poids de polysaccharide, 5—25% en poids d'eau, 5—25% en poids d'émulsifiant, 15—40% en poids de solvants hydrocarbonés, et 1—15% en poids de stabilisant.

8. Emulsion de biopolymère obtenue en soumettant une émulsion eau dans l'huile selon l'une quelconque des revendications précédentes à un traitement de cisaillement ou en broyant dans un broyeur colloïdal ce qui réduit sensiblement de taille des gouttelettes de polysaccharide.

9. Procédé pour concentrer une émulsion selon l'une quelconque des revendications précédentes dans lequel l'eau est éliminée de l'émulsion par évaporation sous pression réduite et à température élevée.

10. Procédé selon la revendication 9 dans lequel l'évaporation est réalisée dans un évaporateur rotatif ou un évaporateur à film étalé.

11. Emulsion de biopolymère concentrée quand elle est produite par un procédé selon la revendication 10.

12. Système aqueux d'imprégnation pour récupération de pétrole épaissi par l'incorporation d'une émulsion selon l'une quelconque des revendications 1—8 ou 11.